# EUROPEAN PATENT SPECIFICATION

(11) **EP 1 957 008 B1**
(45) Date of publication and mention of the grant of the patent: **11.07.2012**
(21) Application number: 05850788.0
(22) Date of filing: 09.12.2005
(51) Int. Cl.: A61F 2/08, A61F 2/00

(54) **IMPLANT FOR TREATING STRESS URINARY INCONTINENCE AND ANTERIOR VAGINAL WALL PROLAPSE**
IMPLANTAT ZUR BEHANDLUNG VON STRESSINKONTINENZ UND PROLAPS DER VORDEREN VAGINALWAND
IMPLANT POUR TRAITER L'INCONTINENCE URINAIRE DE STRESS ET LE PROLAPSUS DE LA PAROI VAGINALE ANTÉRIEURE

(43) Date of publication of application: 20.08.2008
(73) Proprietor: Promedon Do Brasil Produtos Medico-Hospitalares Ltda, 04001-001 Sao Paulo (BR)
(72) Inventor: GRIGUOL, Osvaldo, Nicolas, Cordora (AR)
(74) Representative: Dossmann, Gérard
(86) International application number: PCT/IB2005/004089
(87) International publication number: WO 2007/066169

(56) References cited:
- WO-A-2004/012626
- WO-A-2004/091442
- US-A1- 2003 220 538
- US-A1- 2004 039 453

## Description

The present application relates to the treatment of both stress urinary incontinence and anterior vaginal wall prolapse.

Urinary incontinence impacts at least 20% of healthy middle-aged women. It is important that incontinence symptoms are treated because they impacts not only the physiological, but also the psychological realms of a person's life. Researchers and clinicians are increasingly aware of the importance of identifying urinary incontinence, in particular they are aware that this pathology causes distress or adversely effects health-related quality of life among women.

Anterior vaginal wall prolapse, also called cystocele, is a frequent condition affecting 11% of American women.

The incidence of cystocele doubles each decade of life. In the United States of America, there are 2,9 million of women with grade III and IV cystocele.

The specific treatment for cystocele varies according to several parameters: age, patient's overall health status and medical record, stage of the disease, tolerance to specific medications, procedures or therapies and expectations for the course of the disease.

Treatment may be non-surgical for mild cases or surgical for more severe cystocele cases.

Surgical procedures using a mesh-sling offer better expectations of a permanent vaginal prolapse repair. There are two types of surgical access: vaginal and abdominal (laparoscopic). Laparoscopic procedures require highly skilled professionals, specialized training and a hospital stay of 2-7 days. Vaginal approach has the advantage of being minimally invasive, decreasing postoperative pain and reducing morbidity associated to the abdominal processes.

Different materials have been used to manufacture mesh products, which can be classified into synthetic and biological. Even though synthetic meshes present a higher risk of infection / erosion than biological meshes, the former are preferred due to their mechanical strength and the absence of disease transmission risk. On the other hand, biological materials are absorbed by the body, which requires a new treatment for the recurrence of prolapse.

Different implants have already been disclosed in the prior art.

Patent application WO 00/27304 discloses a suspension device for treating prolapse and urinary incontinence. The device comprises at least one filiform suspension cord with limited elasticity and at least two anchoring parts linked to the ends of the cord.

Patent application US 2004/0039453 describes an implant suitable for addressing one or more disorders including incontinence, cystocele, rectocele, enterocele and prolapse (e.g. uterine and/or vaginal vault). The implant is conformable to irregular surfaces and accommodates different anatomy shapes and sizes. The implant for cystocele is secured in the body without placing tension on the bladder. The implant includes a urethral support portion capable of being placed underneath the patient's urethra, and first and second sling appendages are placed on different sides of the urethra. The sling appendages may be sized and shaped to be secured in the patient's abdominal rectus fascia, or alternatively in the patient's obturator foramen.

Patent application WO03073960 describes an anterior implant adapted to treat central and lateral cystoceles and a single posterior implant for rectocele and hysterocele repairs. The anterior implant includes an inboard area adapted to treat a lateral cystocele and a pair of flanking outboard areas, each adapted to treat a lateral cystocele. After positioning the body of the anterior implant between the patient's bladder and vagina, laterally extending straps are passed through the patient's obturator. The straps function to stabilize the implant. In one embodiment, the anterior implant is provided with a pair of stabilizing straps, one on each side of the implant. In another embodiment, two pairs of stabilizing straps are provided, one pair extending laterally from one side of the anterior implant and another pair extending laterally from the opposite side of the anterior implant.

Patent application WO2004/091443 relates to an implant for the treatment of a cystocele, having a thin and flexible structure, comprising a support body from which extend two anterior suspension harnesses run, arranged to both sides of a sagittal plane, two posterior suspension harnesses, arranged to both sides of the sagittal plane and two medial suspension harnesses arranged to both sides of the sagittal plane and between the anterior and posterior harnesses. The six suspension straps provides distribution of the effort acting on the support body. The anterior suspension straps are inserted in obturators foramen. The middle suspension straps are each placed either in the middle translevator region, or into an obturated foramen in its infero-posterior region. While the posterior straps are either trans-fixed in the uterosacral ligaments or placed across the uterosacral ligaments.

Patent application WO03068107 describes a surgical kit for performing a surgical procedure on a patient to restore a prolapsed organ within a patient's pelvic region. The surgical kit includes a mesh for supporting the organ, the mesh including a support sheet portion to be positioned beneath the organ having a distal end region and a proximal end region, first and second front attachment strips extending from the proximal end region, and first and second rear attachment strips extending from the distal end region. The surgical kit further includes a first guide needle for penetrating tissue within the patient's body to create a passageway through the patient's pelvic region through which the first or second front or rear attachment strips can be pulled.

Patent application WO2004012626 describes a flat implantable device for the surgical treatment of total or partial prolapse of the female pelvic organs or of prolapse of the vaginal vault. The implant has a central body with a trapezoid shape having four arms, two arms coaxial with each other and the other two arms diverging from each other and parallel to the sides of the trapezium. The arms can be anchored, during surgery, to fixed and well identified structures on the patient's pelvis.

Patent application WO2004/098461 describes a prosthesis to be used in the surgical treatment of urogenital prolapse and female urinary incontinence. The prosthesis comprises a mesh with a central body and four arms. The mesh is designed to be placed beneath the bladder under the bladder neck and middle urethra. The first pair of arms extend from each side of the first portion of the central body; said arms are designed to be placed in the obturator foramen bilaterally. The second pair of arms extend from each side of the second portion of the central body; said arms are designed to be placed on the perineum, passing through the levator muscles.

Some of the most frequent postoperative problems that derive from the use of synthetic meshes for treating anterior vaginal wall prolapse are vaginal erosion and mesh shrinkage. The former occurs in 6-16% of the cases, and the latter, in 5-16%. This is confirmed by the following bibliographic references:
- De Tayrac R, et al. Prolapse repair by vaginal route using a new protected low-weight polypropylene mesh: A prospective multicentre study. International Urogynecology Journal - vol. 16 Suppl 2 2005. p. S50 ;
- Cervigni M, et al. Prospective randomized study between two new soft material: Pelvicol and Prolene soft: Preliminary results. International Urogynecology Journal - vol. 16 Suppl 2 2005. p. S84;
- Jacquetin B, et al. Prolene soft mesh for pelvic organ prolapse surgical treatment: A prospective study of 264 patients. IUGA-ICS Meeting Paris. 23-27 August 2004. Abstract N° 767.

Many patients with anterior vaginal wall prolapse also suffer from associated stress urinary incontinence (SUI). Both pathologies are generally treated with one single surgery, by implanting a mesh for prolapse repair and a sling for treating SUI. In the reference below about 687 patients who underwent prolapse surgery, 40,9% underwent a simultaneous stress urinary incontinence surgery.

Cosson M, et al. Prolift mesh (Gynecare) for pelvic organ prolapse surgical treatment using the TVM group technique: A retrospective study of 687 patients. ICS Meeting Montreal. 29 August - 2 Set. 2005. Abstract N° 121.

From this state of the art, it appears that in the recent date many approaches and treatments have been proposed to cure or relieve one or both of these diseases. Nevertheless there remains a very important need for a successful and lasting treatment of both stress urinary incontinence and anterior vaginal wall prolapse.

The problem aimed in the present application is to provide a long lasting, safe, effective treatment of both stress urinary incontinence and anterior vaginal wall prolapse. This problem has been solved by using the implant as described below. The "implant" may also be called "prothesis".

Advantageously, this prothesis is well tolerated by the body, in particular it should be noticed that the vaginal erosion is low. An implant according to the preamble of claim 1 is known from the documents WO-A-2004/091442 and US-A-2003/22 0538.

In a preferred embodiment, the implant consists of a mesh made of non-absorbable synthetic material, in this case, the mesh shrinkage is also low.

Other objects, advantages, features of the present patent application are explained in the following description

A first object of the present invention is an implant (1) for treating both stress urinary incontinence and anterior vaginal wall prolapse comprising :
a central body (2) having a central longitudinal axis (x-x')and comprising a central base (8) and two lateral supporting parts (7) positioned on each side of the central base regard to the central longitudinal axis,
two inferior tapes (6) extending laterally outward from the two lateral supporting parts (7),
two superior tapes (5) extending laterally outward from each side of the central body. The inferior and superior tapes (6,5) are oriented in the direction of the front of the implant, the inferior tapes (6) being adapted to support the bladder and the superior tapes (5) to correct stress urinary incontinence.

This implant is adapted to treat both stress urinary incontinence and anterior vaginal wall prolapse : the inferior and superior tapes (6,5) are adapted to secure the implant and maintain it in the required position, these two pairs of tapes are preferably self-anchoring tapes.

The two superior tapes (5) are designed for the pre-pubic insertion they contribute to reinforce the anterior pubourethral ligaments, thus to stabilize the urethra and consequently to treat the stress urinary incontinence.

The two inferior tapes (6) are destinated to secure the implant, they are adapted to be placed bilaterally in the obturator forament, they contribute to reconstruct the pelvic floor by repairing lateral defects of the bladder and keeping it in its original place.

Once the prothesis has been implanted, the four tapes (5,6) maintain the central body (2) stretched and flat, reducing the possibility of folds.

Further the two lateral supporting parts (7) are designed to laterally support the bladder and thus contribute to correct the lateral defects. The lateral supporting parts (7) have a triangular form.

And the central base (8) is adapted to be placed underneath the bladder and fixed to the vaginal wall. In a prefered embodiment, the form of the central base is essentially rectangular. The central base sustain the bladder and contributes to correct the anterior vaginal wall prolapse and particularly the central defects.

The implant according to the present application is further described below.

Preferably the lengh of the implant is comprised between 280 and 400 mm and the width is comprised between 160 and 230 mm.

The implant according to the present invention allows for cystocele repair as well as stress urinary incontinence repair, which is frequently associated to prolapse, as it was previously mentioned. This is possible due to the implantation of the two superior tapes just above the pubic bone (prepubic). By using this surgical approach technique, intraoperative cystoscopy for discarding bladder perforation can be avoided. This intraoperative cystoscopy is necessary when the tapes are placed beneath the pubic bone (retropubic), as in the prior art. Other implants of the prior art are designed so that the 4 arms are placed in the obturator foramen, in this case the risk for major complications such as obturator nerve and major vessel injury and hemorrhage is increased.

In conclusion, the implant according to the present invention presents the advantage of needing only two transobturator tapes (the other two are prepubic), through a minimally invasive procedure.

Particularly the implant is made in a non-absorbable synthetic material.

The implant is preferably made of polypropylene mesh with specific gravity from 70 to 90 g/m² and break resistance of net from 9 to 12 kg/cm². However, polypropylene meshes with specific gravity from 35 to 120 g/m² and break resistance of net from 6 to 15 kg/cm² can also be used. Other synthetic non-biodegradable type of material, such as polyester, Teflon, nylon, polyethylene terephthalate, polyurethane, and polyamide can also be used for manufacturing the implant. Meshes can also have a silicone or polyurethane coating or they can be coated with substances that favor the rapid integration between mesh and body tissues and reduce the risk of infections, such as collagen, hyaluronic acid, chlorohexidine, heparin, antibiotic delivery systems and silver or other metallic salts.

Preferably, the central base is perforated by holes in order to make the mesh softer, more flexibe and also to diminishe mesh shrinkage.

The mesh of the central base has about 5-40 perforations, preferably 10-25 perforations. Said perforations are preferably symmetrically distributed, following a predetermined pattern, but can also be randomly distributed. The diameter of the perforations varies between 4 and 15 mm and preferably between 5 and 7 mm. The perforations throughout the mesh surface are preferably circular but they can also be oval, rectangular, square, circular or any combination of these shapes; they can also be of varied rhombic shape or of multiple geometric shapes (e.g.: star, etc). Amongst its holes or perforations grows the interconnective tissue between the vaginal flap and the bladder, which leads to a great integration of the implant without a loss of vascularization between the bladder and the vagina. This also contributes to keep the implant stable and fixed. As a matter of fact, since there is normal vascularization in the area of the implant integration, the risk of necrosis and postoperative erosion and also the risk of mesh shrinkage diminishes. Additionally, the holes make the mesh softer and more flexible, which is particularly favorable because it reduces the effect of possible mesh folds that may form during implantation.

The inferior and superior tapes (6,5) are oriented in the direction of the front of the implant.

The two superior tapes, preferably self-anchoring tapes, have an opening angle comprised between 60° and 130° preferably between 90° and 110°. The two inferior tapes, preferably self-anchoring tapes have an opening angle comprised between 135° and 175° preferably between145° and 165°. During implantation of the prothesis, the four self-anchoring tapes are anchored to the tissues, keeping the mesh in place. Subsequently, body tissues grow through the mesh pores, fixing and stabilizing the implant. The best anchorage region of the two superior self-anchoring tapes is the anterior pubourethral ligaments. The best anchorage region of the two inferior self-anchoring tapes is the obturator walls.

The passage of prepubic arms during implantation can be performed with straight needles or with needles similar to those used in pubourethral sling implantation, whereas the passage of transobturator arms is performed with curved C-type or Deschamps needles.

The method for treating both stress urinary incontinence and anterior vaginal wall prolapse is not part of the present invention, the surgical technique is the following:
the patient is placed in the lithotomy position; Allis forceps are applied at the level of the mid urethra and at the lowest part of the cystocele ; a midline incision is made between the two Allis forceps. The cut should be done laterally to the medial edge of the ischio-pubic ramus and inferiorly up to the uterine cervix.

Next mark needle entry points on the suprapubic and vulvar skin. Suprapubic points are marked 5 cm apart at just above of the public bone. The inferior marks are made using the following landmarks: genitofemoral folds at the level of the clitoris, than 3 cm below and 3 cm lateral.

The superior needles are inserted transvaginally in a pre-pubic manner, towards the previously made marks on each side. The superior tapes (5) of the implant are connected to the tip of the needles. Then the needles are pull from the suprapubic region to pass the tapes (5). The zone (3) of the prosthesis must rest on the middle urethra but with no tension.

Next the Deschamps needles are inserted parallel to the ascending ramus of the pubic bone, and turning the wrist and guided by the surgeon index finger, exit through the vaginal incision. After connecting the tip of the needle with the inferior tapes (6), they are pulled through up to the lateral edge of the cystocele.

The mesh should be underneath the cystocele in a tension-free manner and the redundant arms trimmed off. The inferior part (4) of the central body of the implant is then placed underneath the bladder base and sutured with two stitches placed at the vaginal wall.

Vaginal incision is closed using overlap technique to avoid contact of the suture line with the mesh. The vaginal wall is not trimmed unless necessary. The two vaginal flaps are created. One flap is brought over the mesh and sutured to the base of the contralateral flap and its overlying epithelium is superficially cauterized for destruction of all glandular elements. Finally, the remaining flap is sutured over the interposed flap to cover it. A foley catheter and vaginal packing is let in place overnight.

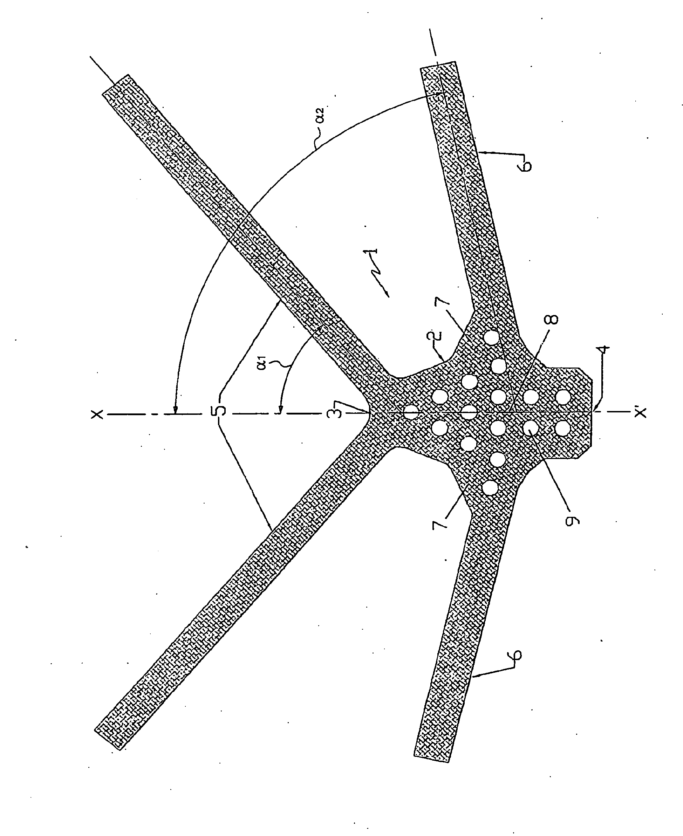

The invention will be better understood with the particular embodiment presented below. This embodiment is not limitative, a form of realization of this particular embodiment is presented in the figure.

An implant according to the present invention, indicated generally with the reference number 1 is presented in the figure, this implant is adapted to treat both stress urinary incontinence and anterior vaginal wall prolapse

In the figure, the implant is represented in a position which is supposed to be vertical.

The implant 1 comprises a central body (2) having a central longitudinal axis (x-x'), with a superior part (3), an inferior part (4) and inferior and superior tapes (5 and 6).

The inferior part (4) of the central body has a general rectangular form which extends along the longitudinal axis (x-x'), the inferior part (4) is continued at its superior end by superior part (3). The superior part (3) has a general triangular form, it is adapted to be placed suburethrally at the mid urethra and designed not to hurt the urethra.

In order to contribute to reinforce the anterior pubourethral ligaments, the superior tapes (5) are symetrically positioned in regard to axis x-x'.

Each the superior tape (5) extend from the superior part (3) of the central body (2) toward the top, in a leant direction in regard to the longitudinal axis (x-x'). The angle of the superior tapes (5) regard to the longitudinal axis (x-x') is avantageously comprised in an angular sector α1 from 30° to 65° preferably from 45° to 55°, this corresponds to an opening angle between the two superior tapes comprised between 60° and 130° preferably between 90° and 110°

The superior tapes (5) are presented in the figure in a general rectangular form.

The two inferior tapes (6) are attached to the central body (2) through two lateral supporting parts (7), the two supporting parts (7) continue each side of the inferior part (4) in a slighly radial manner.

The lateral supporting parts (7) have a general triangular form. Each of the two lateral supporting part is placed in a manner such that the basis (the larger side) of the lateral supporting part is along the side of the inferior part (4).

The lateral supporting parts (7) designed to laterally support the bladder, they are positioned approximatively at the mid distance between the superior end and the inferior end of the central boby (2). The lateral supporting parts (7) are also symetrical in regard to the longitudinal axis (x-x').

The inferior tapes (6) extend in a leant direction in regard to the longitudinal axis (x-x'). The angle of the inferior tapes (6) in regard to the axis (x-x') is avantageously comprised in an angular sector α2 from 67.5° to 87.5° preferably from 72.5° to 82.5°, this corresponds to an opening angle between the two inferior tapes comprised between 135° and 175° preferably between 145° and 165°.

The superior tapes (5) and the two inferior tapes (6) point toward the same general direction (toward the top).

The inferior tapes (6) are also symetrical in regard to longitudinal axis (x-x').

In order to be more flexible, the implant comprises holes which perforate the central body (2) and the supporting parts (7). Sixteen holes (9) are represented, they have a circular section and a diameter comprised between 5 mm and 7mm. The distribution pattern, section of the holes, and size of the holes may be different, e.g. the section may be oval, rectangular, square...

Advantageously, the implant (1) is made of one piece obtain by the molding of a biocompatible synthetic material such as polyester, polypropylene; polyurethane, polyamide, nylon, Teflon, polyethylene terephtalate, or any other thermo-formed plastic.

Advantageously the implant is made of polypropylene with specific gravity from 70 to 90 g/cm² and/or with specific resistance of net from 9 to 12 kg/cm².

Further it is also possible to coat the implant with silicone, polyurethane, collagen, hyaluronic acid, chlorohexidine, heparin, antibiotic delivery systems or silver or other metallic salt.

## Claims

1. Implant (1) for treating both stress urinary incontinence and anterior vaginal wall prolapse comprising :
a central body (2) having a central longitudinal axis and comprising a central base (8) and two lateral supporting parts (7) positioned on each side of the central base in regard to the central longitudinal axis,
two inferior tapes (6) extending laterally outward from the two lateral supporting parts (7), two superior tapes (5) extending laterally outward from each side of the central body, the inferior and superior tapes (6,5) are oriented in the direction of the front of the implant, **characterised in that** the inferior tapes (6) are adapted to support the bladder and the superior tapes (5) to correct stress urinary incontinence and the lateral supporting parts (7) have a triangular form.

2. Implant according to claim 1 **characterised in that** the central base has an essentially rectangular form.

3. Implant according to one of the preceding claims **characterized in that** the two superior tapes have an opening angle comprised between 60° and 130° preferably between 90° and 1 10°.

4. Implant according to one of the preceding claims **characterized in that** the two inferior tapes have an opening angle comprised between 135° and 175° preferably between 145° and 165°.

5. Implant according to one of the preceding claims **characterized in that** the central base is perforated by holes, the section of the holes being selected in the group formed by oval, rectangular, square, circular form or any combination of these forms.

6. Implant according to the preceding claim **characterized in that** the holes are circular with a diameter from 5 to 7 mm.

7. Implant according to the claim 5 or 6 **characterized in that** the central base is perforated by between 10 and 25 holes, the holes being symmetrically or randomly distributed.

8. Implant according to one of the preceding claims **characterized in that** it is made of a biocompatible synthetic material such as polyester, polypropylene, polyurethane, polyamide, nylon, Teflon, polyethylene terephtalate, or any other thermo-formed plastic.

9. Implant according to one of the preceding claims **characterized in that** it is coated with silicone, polyurethane, collagen, hyaluronic acid, chlorohexidine, heparin, antibiotic delivery systems or silver or other metallic salt.

10. Implant according to one of the preceding claims **characterized in that** it is made of polypropylene with specific gravity from 70 to 90 g/cm²

11. Implant according to one of the preceding claims **characterized in that** it is made of polypropylene with specific resistance of net from 9 to 12 kg/cm².

## Revendications

1. Implant (1) destiné au traitement tant d'une incontinence d'urine à l'effort que d'un prolapsus de la paroi vaginale antérieure, comprenant :
- un corps central (2) présentant un axe longitudinal central et comportant une base centrale (8) et deux parties latérales de soutien (7) placées de chaque côté de la base centrale par rapport à l'axe longitudinal central,
- et deux bandes inférieures (6) s'étendant latéralement vers l'extérieur depuis les deux parties latérales de soutien (7), ainsi que deux bandes supérieures (5) s'étendant latéralement vers l'extérieur depuis chaque côté du corps central,
- lesquelles bandes inférieures (6) et supérieures (5) sont orientées en direction de l'avant de l'implant,
**caractérisé en ce que** les bandes inférieures (6) sont adaptées pour soutenir la vessie, et les bandes supérieures (5) pour corriger l'incontinence d'urine à l'effort, et **en ce que** les parties latérales de soutien (7) sont de forme triangulaire.

2. Implant conforme à la revendication 1, **caractérisé en ce que** la base centrale est de forme essentiellement rectangulaire.

3. Implant conforme à l'une des revendications précédentes, **caractérisé en ce que** les deux bandes supérieures font un angle d'ouverture qui vaut de 60° à 130°, et de préférence de 90° à 110°.

4. Implant conforme à l'une des revendications précédentes, **caractérisé en ce que** les deux bandes inférieures font un angle d'ouverture qui vaut de 135° à 175°, et de préférence de 145° à 165°.

5. Implant conforme à l'une des revendications précédentes, **caractérisé en ce que** la base centrale est perforée de trous dont la section est d'une forme choisie parmi les formes ovale, rectangulaire, carrée et circulaire et toute combinaison de ces formes.

6. Implant conforme à la revendication précédente, **caractérisé en ce que** les trous sont de forme circulaire et ont de 5 à 7 mm de diamètre.

7. Implant conforme à la revendication 5 ou 6, **caractérisé en ce que** la base centrale est perforée de 10 à 25 trous, lesquels tous sont répartis de manière symétrique ou aléatoire.

8. Implant conforme à l'une des revendications précédentes, **caractérisé en ce qu'**il est fait d'un matériau synthétique biocompatible, tel un polyester, un polypropylène, un polyuréthane, un polyamide, un nylon, du Téflon, un poly(éthylène téréphtalate) ou toute autre matière plastique thermoformée.

9. Implant conforme à l'une des revendications précédentes, **caractérisé en ce qu'**il est revêtu d'une silicone, d'un polyuréthane, de collagène, d'acide hyaluronique, de chlorhexidine, d'héparine, de systèmes libérant des antibiotiques, ou d'un sel d'argent ou d'un autre métal.

10. Implant conforme à l'une des revendications précédentes, **caractérisé en ce qu'**il est fait d'un polypropylène présentant un poids spécifique de 70 à 90 g/cm².

11. Implant conforme à l'une des revendications précédentes, **caractérisé en ce qu'**il est fait d'un polypropylène présentant une résistance spécifique de filet de 9 à 12 kg/cm².

## Patentansprüche

1. Implantat (1) zur Behandlung von sowohl stressbedingter Harninkontinenz als auch eines Prolaps der vorderen Vaginalwand, umfassend:
einen mittleren Körper (2) mit einer Längsmittelachse und mit einer mittigen Basis (8) und zwei seitlichen Stützteilen (7), die auf jeder Seite der mittigen Basis im Hinblick auf die Längsmittelachse positioniert sind,
zwei untere Bänder (6), die sich von den zwei seitlichen Stützteilen (7) seitwärts nach außen erstrecken, wobei sich zwei obere Bänder (5) von jeder Seite des mittigen Körpers seitwärts nach außen erstrecken und die unteren und oberen Bänder (6, 5) in Richtung der Vorderseite des Implantats ausgerichtet sind,
**dadurch gekennzeichnet, dass** die unteren Bänder (6) dazu ausgelegt sind, die Blase zu stützen und die oberen Bänder (5) dazu ausgelegt sind, stressbedingte Harninkontinenz zu korrigieren und die seitlichen Stützteile (7) eine dreieckige Form haben.

2. Implantat nach Anspruch 1, **dadurch gekennzeichnet, dass** die mittige Basis eine im Wesentlichen rechteckige Form hat.

3. Implantat nach einem der vorhergehenden Ansprüche, **dadurch gekennzeichnet, dass** die zwei oberen Bänder einen Öffnungswinkel im Bereich zwischen 60° und 130°, vorzugsweise zwischen 90° und 110° haben.

4. Implantat nach einem der vorhergehenden Ansprüche, **dadurch gekennzeichnet, dass** die zwei unteren Bänder einen Öffnungswinkel im Bereich zwischen 135° und 175°, vorzugsweise zwischen 145° und 165° haben.

5. Implantat nach einem der vorhergehenden Ansprüche, **dadurch gekennzeichnet, dass** die mittige Basis durch Löcher perforiert ist, wobei der Querschnitt der Löcher aus der von einer ovalen, rechteckigen, quadratischen, kreisrunden Form gebildeten Gruppe oder aus einer Kombination dieser Formen ausgewählt ist.

6. Implantat nach dem vorhergehenden Anspruch, **dadurch gekennzeichnet, dass** die Löcher kreisrund sind mit einem Durchmesser von 5 bis 7 mm.

7. Implantat nach Anspruch 5 oder 6, **dadurch gekennzeichnet, dass** die mittige Basis durch zwischen 10 und 25 Löcher perforiert ist, wobei die Löcher symmetrisch oder zufällig verteilt sind.

8. Implantat nach einem der vorhergehenden Ansprüche, **dadurch gekennzeichnet, dass** es aus einem biokompatiblen synthetischen Material wie zum Beispiel Polyester, Polypropylen, Polyurethan, Polyamid, Nylon, Teflon, Polyethylenterephthalat oder einem anderen thermogeformten Kunststoff besteht.

9. Implantat nach einem der vorhergehenden Ansprüche, **dadurch gekennzeichnet, dass** es mit Silicon, Polyurethan, Kollagen, Hyaluronsäure, Chlorhexidin, Heparin, Verabreichungssystemen für Antibiotika oder Silber oder einem anderen Metallsalz beschichtet ist.

10. Implantat nach einem der vorhergehenden Ansprüche, **dadurch gekennzeichnet, dass** es aus Polypropylen mit einem spezifischen Gewicht von 70 bis 90 g/cm² besteht.

11. Implantat nach einem der vorhergehenden Ansprüche, **dadurch gekennzeichnet, dass** es aus Polypropylen mit einem spezifischen Widerstand des Netzes von 9 bis 12 kg/cm² besteht.
